# EUROPEAN PATENT APPLICATION

(11) **EP 4 414 353 A1**
(43) Date of publication of application: **14.08.2024**
(21) Application number: 23155916.2
(22) Date of filing: 09.02.2023
(51) Int. Cl.: C07C 41/16, C07C 43/11

(54) **PROCESS FOR PREPARING DIPOPYLENE GLYCOL ISOMERS**

(71) Applicant: Oleon N.V., 9940 Ertvelde (BE)
(72) Inventor: VAN HOLEN, Jurgen, 9400 NINOVE (BE); VAN VAERENBERGH, Beau, 1770 LIEDEKERKE (BE)
(74) Representative: Santarelli

(57) **Abstract**

The present invention relates to a process for preparing a new mixture of dipropylene glycol isomers, and to the uses of said mixture in various fields.

## Description

The present invention relates to a new process for preparing a mixture of dipropylene glycol isomers, to this new mixture, and to the use of the later in various fields.

Dipropylene glycol, also named oxydipropanol; is usually a mixture of isomers. Indeed, current commercially available dipropylene glycol is a mixture of three isomers, comprising typically:
- 40-60% by weight of 2-(2-hydroxypropoxy)-1-propanol;
- 30-45% by weight of 1,1'-oxydi-2-propanol;
- less than 10% by weight of 2,2'-oxydipropanol;
weight percentages being based on the weight of the mixture of dipropylene glycol isomers.

Nowadays, dipropylene glycol is industrially obtained as a by-product of the monopropylene glycol production, by hydrolysis of propylene oxide. This process is energy intensive, as it is typically run at 200°C and 15 bar of pressure. Propylene oxide is a toxic chemical and highly volatile (boiling point 35°C).

In the patent application WO2012/154460, a process for producing bioderived dipropylene and tripropylene glycols through an acid-catalyzed condensation of bioderived propylene glycol at elevated temperature is disclosed. Besides the dipropylene and tripropylene glycols, other chemicals, such as 4-methyl-2-ethyl-1, 3-dioxolane and propanal are also formed. In Examples, the dipropylene glycol contents represent at most 12.35 wt% of the reaction product mixture.

Thus, there is a need for an improved process for preparing dipropylene glycol.

The Applicant developed a new process for preparing dipropylene glycol, which is less energy consuming, do not require the use of toxic raw material and which leads to a good yield.

Accordingly, the present invention relates to a process for preparing a mixture of dipropylene glycol isomers comprising a step of reacting 4-methyl-1,3-dioxolan-2-one with propane-1,2-diol.

The mixture of dipropylene glycol isomers comprises 1,1'-oxydi-2-propanol, 2-(2-hydroxypropoxy)-1-propanol, and optionally 2,2'-oxydipropanol.

1,1'-oxydi-2-propanol, (CAS-No.: 110-98-5), also named 2,2'-dihydroxydipropyl-ether, is of Formula I:

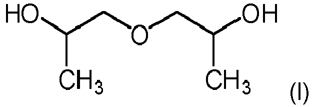

2-(2-hydroxypropoxy)-1-propanol, (CAS-No.: 106-62-7), also named 2-hydroxypropyl-2'-hydroxyisopropylether is of Formula II:

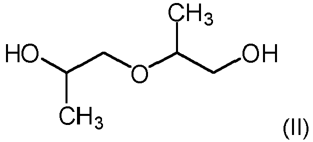

2,2'-oxydipropanol, (CAS-No.: 108-61-2), is of Formula (III):

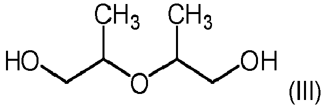

4-methyl-1,3-dioxolan-2-one is also named propylene carbonate.

Propane-1,2-diol is also named propylene glycol or monopropylene glycol.

Preferably, the molar ratio propane-1,2-diol / 4-methyl-1,3-dioxolan-2-one is of at least 1.

In particular, the molar ratio propane-1,2-diol / 4-methyl-1,3-dioxolan-2-one is comprised between 1 and 5, preferably, between 1 and 3.

In the present application, unless otherwise indicated, all ranges of values used are to be understood as being inclusive limits.

Advantageously, in the process according to the invention, the reaction is conducted in the presence of a basic catalyst.

The basic catalyst may be homogeneous or heterogeneous.

In particular, the basic catalyst is an alkali metal hydroxide, alkali metal methoxide, an alkali metal carbonate, an alkali metal hydrogen carbonate, sodium aluminate or magnesium oxide.

More particularly, the alkali metal hydroxide is sodium hydroxide, lithium hydroxide, potassium hydroxide, barium hydroxide and/or magnesium hydroxide.

More particularly, the alkali metal methoxide is sodium methoxide.

More particularly, the alkali metal carbonate is potassium carbonate, sodium carbonate, lithium carbonate, barium carbonate or calcium carbonate.

Preferably, the quantity of basic catalyst is of at least 0.01% by weight, more preferably of at least 0.05% by weight, even more preferably of at least 0.1% by weight based on the weight of propane-1,2-diol.

The reaction is preferably conducted under stirring and heating.

Advantageously, in the process according to the invention, the reaction is conducted at a temperature of at least 100°C.

Preferably, the reaction is conducted at a temperature of at least 120°C, even more preferably at least 140°C. In particular, the reaction is carried out at a temperature comprised between 100 and 200°C, preferably between 120 and 180°C.

Preferably, the reaction mixture is free of water.

Preferably, the reaction is carried out until the 4-methyl-1,3-dioxolan-2-one disappeared. The disappearance of 4-methyl-1,3-dioxolan-2-one may be controlled by gas chromatography or by infrared.

The unreacted propane-1,2-diol may be recovered, preferably by distillation.

The unreacted propane-1,2-diol may be recycled, e.g. reused in a subsequent implementation of the process according to the invention.

The process according to the invention is a process economically viable. Indeed, the process according to the invention makes it possible to obtain a reaction product wherein the mixture of dipropylene isomers represents at least 25% by weight, preferably at least 35% by weight based on the weight of the reaction product.

Advantageously, the process according to the invention, further comprises a step of purification of the mixture of dipropylene glycol isomers.

Preferably, the purification is achieved by distillation, such as by molecular distillation, short path distillation or directly from the reactor.

Prior to the purification step and after the reaction step, there may be a step of separation of the basic catalyst.

Advantageously, in the process according to the invention, the 4-methyl-1,3-dioxolan-2-one is prepared by reacting propane-1,2-diol with dimethyl carbonate.

Preferably, the reaction between propane-1,2-diol with dimethyl carbonate is conducted in the presence of a base such as sodium methoxide.

Preferably, the reaction between propane-1,2-diol with dimethyl carbonate is conducted at a temperature of at least 60°C, more preferably of at least 70°C, even more preferably of at least 80°C.

The methanol formed during the reaction may be removed by distillation.

Advantageously, in the process according to the invention, the propane-1,2-diol is obtained from a renewable resource.

Preferably, the renewable resource is a plant, in particular a vegetable oil.

More particularly, the propane-1,2-diol is obtained from glycerol.

Thus, the resulting mixture of dipropylene glycol isomers may contain at least 50% of renewable carbon, the determination of the percentage of renewable carbon being made in accordance with ASTM D 6866.

In a preferred embodiment of the process according to the invention, propane-1,2-diol obtained from a renewable resource reacts with 4-methyl-1,3-dioxolan-2-one prepared from propane-1,2-diol obtained from a renewable resource.

Thus, the resulting mixture of dipropylene glycol isomers contain at least 90% of renewable carbon, the determination of the percentage of renewable carbon being made in accordance with ASTM D 6866.

The process according to the invention can be a batch, a semi-batch, or a continuous process.

Surprisingly, the content of dipropylene glycol isomers obtained from the process according to the invention is different from the content of isomers usually obtained with other known processes.

The present invention also relates to a mixture of dipropylene glycol isomers comprising at least 60% by weight of 1,1'-oxydi-2-propanol and between 15% and 40% by weight of 2-(2-hydroxypropoxy)-1-propanol, weight percentages being based on the weight of the mixture of dipropylene glycol isomers.

The 1,1'-oxydi-2-propanol and 2-(2-hydroxypropoxy)-1-propanol are as described above.

Preferably, the quantity of 1,1'-oxydi-2-propanol is of at least 65% by weight, more preferably of at least 70% by weight based on the weight of the mixture of dipropylene glycol isomers.

Preferably, the quantity of 1,1'-oxydi-2-propanol is of at most 85% by weight, more preferably of at most 80% by weight based on the weight of the mixture of dipropylene glycol isomers.

Preferably, the quantity of 2-(2-hydroxypropoxy)-1-propanol is comprised between 20% and 40% by weight, more preferably between 20% and 35% by weight, even more preferably between 20% and 30% based on the weight of the mixture of dipropylene glycol isomers.

Advantageously, the mixture of dipropylene glycol isomers according to the invention, further comprises 2,2'-oxydipropanol.

2,2'-oxydipropanol is as described above.

Preferably, the quantity of 2,2'-oxydipropanol is of at most 5% by weight, more preferably of at most 3% by weight, even more preferably of at most 2% by weight based on the weight of the mixture of dipropylene glycol isomers.

Preferably, the quantity of 2,2'-oxydipropanol is of at least 0.05% by weight, more preferably of at least 0.1% by weight, even more preferably of at least 0.2% by weight based on the weight of the mixture of dipropylene glycol isomers.

In a preferred embodiment, the mixture of isomers of dipropylene glycol according to the invention, comprises:
- 65-80% by weight of 1,1'-oxydi-2-propanol;
- 20-35% by weight of 2-(2-hydroxypropoxy)-1-propanol;
- at most 5% by weight of 2,2'-oxydipropanol;
weight percentages being based on the weight of the mixture dipropylene glycol isomers.

In a particularly preferred embodiment, the mixture of isomers of dipropylene glycol according to the invention, comprises:
- 70-80% by weight of 1,1'-oxydi-2-propanol;
- 20-30% by weight of 2-(2-hydroxypropoxy)-1-propanol;
- at most 2% by weight of 2,2'-oxydipropanol;
weight percentages being based on the weight of the mixture of dipropylene glycol isomers.

Advantageously, in the mixture of dipropylene glycol isomers according to the invention, the dipropylene glycol isomers contain at least 50% of renewable carbon, the determination of the percentage of renewable carbon being made in accordance with ASTM D 6866.

The determination of the percentage of renewable carbon, also named degree of renewability or biobased content, can preferably be carried out according to the standard ASTM D 6866, by measuring the level of carbon 14 present in the product. A molecule obtained from a renewable resource (from a plant, an animal or an alga) contains a characteristic quantity of carbon 14, which distinguishes it from products obtained from fossil resources which do not contain carbon 14.

Preferably, in the mixture of dipropylene glycol isomers according to the invention, the dipropylene glycol isomers contain at least 70% of renewable carbon, more preferably at least 90% of renewable carbon, the determination of the percentage of renewable carbon being made in accordance with ASTM D 6866.

Advantageously, the process according to the invention makes it possible to obtain mixtures of dipropylene glycol isomers according to the invention. Thus, the mixture of dipropylene glycol isomers according to the invention is obtainable by the process according to the invention.

The present invention also concerns the use of the mixture of dipropylene glycol isomers according to the invention, as a solvent.

More particularly, the mixture of dipropylene glycol isomers is used as solvent in cosmetic products, fragrances, pesticides, deicers, inks, and/or lubricants.

The present invention also concerns the use of the mixture of dipropylene glycol isomers according to the invention, as a reactive intermediate in the preparation of plasticizers, polymers and/or resins.

More particularly, the mixture of dipropylene glycol isomers can be used as a polyol, preferably in the preparation of polymers, such as polyurethanes, or resins, such as polyester or alkyd resins.

More particularly, the mixture of dipropylene glycol isomers can be used as a phtalate alternative, preferably in the preparation of plasticizers.

The invention is further described in the following examples. It will be appreciated that the invention as claimed is not intended to be limited in any way by these examples.

### Example 1: Process for preparing mixtures of dipropylene glycol isomers according to the invention

Three mixtures of dipropylene glycol isomers according to the invention, DPG1, DPG2 and DPG3 were prepared using propane-1,2-diol and 4-methyl-1,3-dioxolan-2-one.

### 1.1 DPG1

DPG1 was prepared using 1 mol of propane-1,2-diol and 1 mol of 4-methyl-1,3-dioxolan-2-one.

In a five-necked flask equipped with a stirrer, a thermometer, a dropping funnel, a nitrogen introducing tube and a reflux condenser, 1 mol (76.1 g) of propane-1,2-diol (Radianol 4710 from Oleon) and 0.045 mol (1.8 g) of sodium hydroxide were charged and dissolved by heating to 70°C. Next, 1 mol (102.1 g) of 4-methyl-1,3-dioxolan-2-one (Jeffsol propylene carbonate from Huntsman) was added dropwise at 140°C over 5 hours while stirring and blowing nitrogen. After completion of the dropwise addition, the mixture was stirred at the same temperature for an additional 5 hours.

Gas chromatography analysis was done on an Agilent column CP9106 (30 meter) with an FID detector.

As a result of gas chromatography analysis of this reaction product, unreacted monopropylene glycol represented 31.9%, dipropylene glycol represented 53%, and tripropylene glycol represented 15.1%, percentages being based on the total area represented by the reaction product on the chromatogram.

The dipropylene glycol was further purified by distillation under vacuum.

### 1.2 DPG2

DPG2 was prepared using 3 mol of propane-1,2-diol and 1 mol of 4-methyl-1,3-dioxolan-2-one. The method is the same as the one described above.

A gas chromatography analysis of this reaction product was performed with the same material as described above.

Unreacted propane-1,2-diol represented 67.7%, dipropylene glycol represented 30.3%, and tripropylene glycol represented 2%, percentages being based on the total area represented by the reaction product on the chromatogram.

The dipropylene glycol was further purified by distillation under vacuum.

### 1.3 DPG3

DPG3 was prepared using propane-1,2-diol and 4-methyl-1,3-dioxolan-2-one, wherein the 4-methyl-1,3-dioxolan-2-one was prepared *in situ.*

228.3 g of propane-1,2-diol (Radianol 4710 from Oleon) and 135.2 g of dimethyl carbonate (Daxsol dimethyl carbonate from UBE) were introduced into a four-necked flask equipped with a stirrer, a thermometer, a nitrogen introducing tube and a reflux condenser. Then, 0.228 g of sodium methoxide (Sigma Aldrich) was added and dissolved by heating to 70°C. The mixture was stirred and heated to 80°C until all dimethyl carbonate has reacted (controlled by gas chromatography). The methanol formed was removed from the mixture through distillation.

The reaction temperature was increased to 140°C while stirring and blowing nitrogen until all 4-methyl-1,3-dioxolan-2-one has reacted (controlled by gas chromatography).

A gas chromatography analysis of this reaction product was performed with the same material as described above.

Unreacted propane-1,2-diol represented 36.1%, dipropylene glycol represented 49.7%, and tripropylene glycol represented 9.4%, percentages being based on the total area represented by the reaction product on the chromatogram.

The dipropylene glycol was further purified by distillation under vacuum.

### Example 2: Analysis of the mixture of dipropylene glycol isomers according to the invention

The identification and quantification of isomers of dipropylene glycol obtained in Example 1, was determined by gas chromatography analysis performed on an Agilent column CP9106 (30 meter) with an FID detector.

Results are gathered in Table 1 below:

**Table 1: Content of mixtures of dipropylene glycol isomers DPG1 and DPG2**

| | DPG1 (wt%) | DPG2 (wt%) | DPG3 (wt%) |
|---|---|---|---|
| 1,1'-oxydi-2-propanol | 75.9 | 73.7 | 78.2 |
| 2-(2-hydroxypropoxy)-1-propanol | 23.6 | 25.5 | 21.0 |
| 2,2'-oxydipropanol | 0.5 | 0.8 | 0.8 |

## Claims

1. Process for preparing a mixture of dipropylene glycol isomers comprising a step of reacting 4-methyl-1,3-dioxolan-2-one with propane-1,2-diol.

2. Process according to claim 1, wherein the reaction is conducted in the presence of a basic catalyst.

3. Process according to claim 1 or 2, wherein the reaction is conducted at a temperature of at least 100°C.

4. Process according to any of claims 1 to 3, further comprising a step of purification of the mixture of dipropylene glycol isomers.

5. Process according to any of claims 1 to 4, wherein the 4-methyl-1,3-dioxolan-2-one is prepared by reacting propane-1,2-diol with dimethyl carbonate.

6. Process according to any of claims 1 to 5, wherein the propane-1,2-diol is obtained from a renewable resource.

7. Mixture of dipropylene glycol isomers comprising at least 60% by weight of 1,1'-oxydi-2-propanol and between 15% and 40% by weight of 2-(2-hydroxypropoxy)-1-propanol, weight percentages being based on the weight of the mixture of dipropylene glycol isomers.

8. Mixture of dipropylene glycol isomers according to claim 7, further comprising 2,2'-oxydipropanol.

9. Mixture of dipropylene glycol isomers according to claim 7 or 8, wherein the dipropylene glycol isomers contain at least 50% of renewable carbon, the determination of the percentage of renewable carbon being made in accordance with ASTM D 6866.

10. Use of the mixture of dipropylene glycol isomers according to any of claims 7 to 9, as a solvent.

11. Use of the mixture of dipropylene glycol isomers according to any of claims 7 to 9, as a reactive intermediate in the preparation of plasticizers, polymers and/or resins.
